# EUROPÄISCHE PATENTANMELDUNG

(11) **EP 1 340 491 A2**
(43) Veröffentlichungstag der Anmeldung: **03.09.2003**
(21) Anmeldenummer: 03002420.2
(22) Anmeldetag: 05.02.2003
(51) Int. Cl.: A61K 7/48, A61K 7/00

(54) **Wasser-in-Öl-Emulsion zur Gesichtsreinigung**

(30) Priorität: 21.02.2002 DE 10207269
(71) Anmelder: Beiersdorf AG, 20245 Hamburg (DE)
(72) Erfinder: Kröpke, Rainer, 22869 Schenefeld (DE); Nielsen, Jens, 24558 Henstedt-Ulzburg (DE)

(57) **Zusammenfassung**

Kosmetische und/oder dermatologische Wasser-in-Öl-Emulsionen, die auf der Haut aufbrechen, enthaltend
a) eine wässrige Phase mit einem Anteil von mindestens 75 Gewichts-%
b) eine lipophile Phase mit einem Anteil von höchstens 20 Gewichts-% und
c) ein oder mehrere Hydrocolloide aus der Gruppe der Gummen, Polysaccharide, Cellulosederivate, Schichtsilikate, Polyacrylate und/oder anderen Polymeren mit einem Anteil von 0,1 bis 5 Gewichts-%,
d) einen W/O Emulgator oder ein Gemisch aus mehreren W/O Emulgatoren, gewählt aus der Gruppe der grenzflächenaktiven Substanzen der allgemeinen Struktur A-B-A', wobei A und A' gleiche oder verschiedenen hydrophobe organische Reste darstellen und B eine hydrophile Gruppe bedeutet, in einer Konzentration von 1 bis 8 Gewichts-% jeweils bezogen auf das Gesamtgewicht der Emulsion, mit einem Verhältnis von Hydrocolloiden zu W/O-Emulgatoren von 0,25:1 bis 1:1.

## Beschreibung

Die vorliegende Erfindung betriffi auf der Haut aufbrechende kosmetische und/oder dermatologische Wasser-in-Öl-Emulsionen (W/O-Emulsionen), ihre Anwendung insbesondere zur Gesichtsreinigung sowie mit dieser Emulsion getränkte unlösliche Substrate.

Im Bereich der dekorativen kosmetik finden eine Vielzahl von unterschiedlichen Stoffen Anwendung. Als Farbstoffe werden neben anorganischen Pigmenten wie Silikaten [Magnesiumsilikat (Talkum), Aluminiumsilikat (kaolin)] und Metalloxiden (Chrom-, Eisen-, Mangan-, Titan- und Zinkoxiden) organische Farbpigmente eingesetzt. Als Bindemittel finden unter anderem Stearinsäureester, Lanolinalkohol und -acetat Verwendung. In vielen Formulierungen werden Wachse wie Bienenwachs oder Carnaubawachs und Öle wie Paraffinöle, Silikonöle oder Ricinusöl eingesetzt. Des weiteren können dekorative Kosmetika Konservierungssioffe, Antipxidantien, Verdickungsmittel und andere Zusätze enthalten.

Um diese Vielzahl völlig unterschiedlicher Stoffe von der Haut zu entfernen, bedarf es entsprechender kosmetischer Reinigungsmittel. Sie müssen unpolare Verbindungen wie Wachse, Öle und Silikonverbindungen lösen und gleichzeitig die schwerlöslichen Pigrnente wie Talkum oder Titandioxid aufnehmen. Dies gilt insbesondere für Wimpemtüsche, Mascara, Lidschatten und Kajalstifte. Andererseits . müssen sie so hautverträglich wie möglich sein, um bei den Anwendern keine Hautrötungen oder Schleimhautimtationen auszulösen. Üblicherweise werden Zubereitungen, wie sie beispielsweise in der EP 0705592 B1 offenbart sind, entweder in Form von Lotionen, gegebenenfalls auf Watteträgern, oder als Cremes angeboten. Da es sich stets um Öl-in-Wasser-Emulsionen handelt, bleibt bei diesen Produkten oft ein leicht schmieriges Hautgefühl zurück, was vermutlich auf die Ölkomponenten der Reinigungsemulsion zurückzuführen ist. Des Weiteren enthalten diese Produkte üblicher Weise Tenside.

Diese Substanzen sind in der Regel jedoch nicht besonders hautfreundlich und können, wenn sie beispielsweise beim Abschminken von Lidschatten, Mascara oder "Eye-Linern" in die Augen geraten, zu starken Schleimhautreizungen führen.

Es war deshalb die Aufgabe der vorliegenden Erfindung, den genannten Mängeln des Standes der Technik abzuhelfen und (schleim-) hautfreundliche Reinigungsemulsionen zu entwickeln, die keinen Fetffilm auf der Haut zurück lassen.

Völlig überraschend und für den Fachmann nicht vorhersehbar gelingt es mit kosmetischen und/oder dermatologischen Wasser-in-Öl-Emulsionen, die auf der Haut aufbrechen, enthaltend
a) eine wässrige Phase mit einem Anteil von mindestens 75 Gewichts-%
b) eine lipophile Phase mit einem Anteil von höchstens 20 Gewichts-% und
c) ein oder mehrere Hydrocolloide aus der Gruppe der Gummen, Polysaccharide, Cellulosederivate, Schichtsilikate, Polyacrylate und/oder anderen Polymeren mit einem Anteil von 0,1 bis 5 Gewichts-%,
d) einen W/O Emulgator oder ein Gemisch aus mehreren W/O Emulgatoren, gewählt aus der Gruppe der grenzflächenaktiven Substanzen der allgemeinen Struktur A-B-A',
wobei A und A' gleiche oder verschiedenen hydrophobe organische. Reste darstellen und B eine hydrophile Gruppe bedeutet, in einer Konzentration von 1 bis 8 Gewichts-% jeweils bezogen auf das Gesamtgewicht der Emulsion,
mit einem Verhältnis von Hydrocolloiden zu W/O-Emulgatoren von 0,25:1 bis 1:1, den Mängeln des Standes der Technik abzuhelfen.

Unter Aufbrechen im Sinne der Erfindung versteht man dabei eine Art Umkehr der Emulsion in eine Öl-in-Wasser-Emulsion (O/W-Emulsion) auf der Haut. Bei diesem Prozess werden die Schmutz partikel von der Haut abgelöst und können einfach mit Wasser oder einem unlöslichen Substrat wie zum Beispiel einem Tuch und/oder Watte entfernt werden. Da bei diesem Reinigungsverfahren auf Tenside verzichtet werden kann, verhalten sich die erfindungsgemäßen Emulsionen außerordentlich haut- und schleimhautfreundlich und lassen keinen für den Verbraucher so unangenehmen Fetttilm auf der Haut zurück.

Der Zeitpunkt des Aufbrechens der erfindungsgemäßen Emulsionen lässt sich durch die Konzentration an Wasser und gegebenenfalls durch Zusatz mindestens eines Hydrocolloids einstellen, wobei durch Verringerung des Wasseranteils und/oder die Erhöhung des Hydrocolloidanteils die Zeitdauer bis zum Aufbrechen der Emulsion verlängert wird.

Zwar sind Wasser-in-Öl-Emulsionen mit einem einem Gehalt an wässriger Phase von über 70 Gewichts-% an sich bekannt (sogenannte "high internal phase emulsions"). Derartige Produkte werden bisher jedoch nur Pflegezubereitungen angeboten, die längere Zeit auf der Haut verbleiben (sogenannte "leave-on"-Produkte; z.B. DE 198 54 497, DE 199 24 276). Ferner sind solche Emulsionen. in Kombination mit Polymerschäumen für Windeln bekannt (US 5,741,581; US 5,650,222; 5,744,506). Diese Schriften konnten jedoch nicht den Weg zur vorliegenden Erfindung weisen.

Erfindungsgemäß enthalten als Emulsionen vorliegenden Zubereitungen insbesondere vorteilhaft ein oder mehrere Hydrocolloide. Diese Hydrocolloide können vorteilhaft gewählt werden aus der Gruppe der Gummen, Polysaccharide, Cellulosederivate, Schichtsilikate, Polyacrylate und/oder anderen Polymeren.

Erfindungsgemäß enthalten als Hydrogele vorliegenden Zubereitungen ein oder mehrere Hydrocolloide. Diese Hydrocolloide können vorteilhaft aus der vorgenannten Gruppe gewählt werden.

Zu den erfindungsgemäß vorteilhaften Gummen zählt man Pflanzen- oder Baumsäfte, die an der Luft erhärten und Harze bilden oder Extrakte aus Wasserpflanzen. Aus dieser Gruppe können vorteilhaft im Sinne der vorliegenden Erfindung gewählt werden beispielsweise Gummi Arabicum, Johannisbrotmehl, Tragacanth, Karaya, Guar Gummi, Pektin, Gellan Gummi, Carrageen, Agar, Algine, Chondrus, Xanthan Gummi.

Weiterhin vorteilhaft ist die Verwendung von derivatisierten Gummen wie z.B. Hydroxypropyl Guar (Jaguar® HP 8).

Zu den erfindungsgemäß vorteilhaften Polysacchariden und -derivaten zählen unter anderem Hyaluronsäure, Chitin und Chitosan, Chondroitinsulfate, Stärke und Stärkederivate.

Zu den erfindungs gemäß vorteilhaften Cellulosederivaten zählen unter anderem Methylcellulose, Carboxymethylcellulose, Hydroxyethylcellulose, Hydroxypropylmethylcellulose.

Unter den erfindungsgemäß voteilhaften Schichtsilikaten befinden sich natürlich vorkommende und synthetische Tonerden wie z.B. Montmorillonit, Bentonit, Hektorit, Laponit, Magnesiumaluminiumsilikate wie Veegum®. Diese können als solche oder in modifizierter Form verwendet werden wie z.B. Stearylalkonium Hektorite.

Weiterhin können vorteilhaft auch Kieselsäuregele verwendet werden.

Unter den erfindungsgemäß bevorzugten Polyacrylaten befinden sich z.B. Carbopol Typen der Firma Goodrich (Carbopol 980, 981,1382, 5984, 2984, EDT 2001 oder Pemulen TR2).

Unter den erfindungsgemäß bevorzugten Polymeren befinden sich z.B. Polyacrylamide (Seppigel 305), Polyvinylalkohole, PVP, PVP / VA Copolymere, Polyglycole.

Sie werden in einer Konzentration von 0,01 bis 10,0 Gewichts-% und insbesondere in einer Konzentration von 0,1 bis 5,0 Gewichts-%, jeweils bezogen auf das Gesamtgewicht der Emulsion eingesetzt.

Die erfindungsgemäßen W/O-Emulgatoren werden gewählt aus der Gruppe der Substanzen der allgemeinen Formel 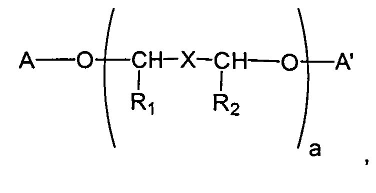 wobei
- A und A' gleiche oder verschiedene hydrophobe organische Reste darstellen,
- a eine Zahl von 1 bis 100, vorzugsweise 2 bis 60, insbesondere 5 bis 40 darstellt,
- X eine Einfachbindung oder die Gruppe 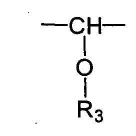
- darstellt,
- R₁ und R₂ unabhängig voneinander so gewählt werden H, Methyl, dass aber nicht beide Reste gleichzeitig Methyl darstellen,
- R₃ gewählt wird aus der Gruppe H, sowie der verzweigten und unverzweigten, gesättigten und ungesättigten Alkyl- und Acylreste mit 1 - 20 Kohlenstoffatomen,
oder dass der oder die W/O-Emulgatoren gewählt werden aus der Gruppe der Fettalkohole mit 8 - 30 Kohlen stoffatomen, Monoglycerinester gesättigter und/oder ungesättigter, verzweigter und/oder unverzweigter Alkancarbonsäuren einer kettenlänge von 8 - 24, insbesondere 12 - 18 C-Atomen, Diglycennester gesättigter und/oder ungesättigter, verzweigter und/oder unverzweigter Alkancarbonsäuren einer Kettenlänge von 8 - 24, insbesondere 12 - 18 C-Atomen, Triglycerinester gesättigter und/oder ungesättigter, verzweigter und/oder unverzweigter Alkancarbonsäuren einer Kettenlänge von 8 - 24, insbesondere 12 - 18 C-Atomen, Polyglycerinester gesättigter und/oder ungesättigter, verzweigter und/oder unverzweigfer Alkancarbonsäuren einer kettenlänge von 8 - 24, insbesondere 12 - 18 C-Atomen mit bis zu 10 Glycenneinheiten, Monoglyceririether gesättigter und/oder ungesättigter, verzweigter und/oder unverzweigter Alkohole einer Kettenlänge von 8 - 24, insbesondere 12 - 18 C-Atomen, Diglycennether gesättigter und/oder ungesättigter, verzweigter und/oder unverzweigter Alkohole einer Kettenlänge von 8 - 24, insbesondere 12 - 18 C-Atomen, Triglycerinether gesättigter und/oder ungesättigter, verzweigter und/oder unverzweigter Alkohole einer Kettenlänge von 8 - 24, insbesondere 12 - 18 C-Atomen, Polyglycerinether gesättigter und/oder ungesättigter, verzweigter und/oder unverzweigter Alkancarbonsäuren einer kettenlänge von 8 - 24, insbesondere 12 - 18 C-Atomen mit bis zu 10 Glycerineinheiten, Propylenglycolester gesättigter und/oder ungesättigter, verzweigter und/oder unverzweigter Alkancarbonsäuren einer Kettenlänge von 8 - 24, insbesondere 12 - 18 C-Atomen, Sorbitanester gesättigter und/oder ungesättigter, verzweigter und/oder unverzweigter Alkancarbonsäuren einer Kettenlänge von 8 - 24, insbesondere 12 - 18 C-Atomen, Sorbitanester von Polyolen, insbesondere des Glycerins, Pentaerythritylester gesättigter und/oder ungesättigter, verzweigter und/oder unverzweigter Alkancarbonsäuren einer Kettenlänge von 8 - 24, insbesondere 12 - 18 C-Atomen, Methylglucose Ester gesättigter und/oder ungesättigter, verzweigter und/oder unverzweigter Alkancarbonsäuren einer Kettenlänge von 8 - 24, insbesondere 12 - 18 C-Atomen, Polyglycerin Methylglucose Ester gesättigter und/oder ungesättigter, verzweigter und/oder unverzweigter Alkancarbonsäuren einer Kettenlänge von 8 - 24, insbesondere 12 - 18 C-Atomen oder dass die vorstehend genannten Typen von W/O-Emulgatoren zusätzlich in der Weise polyethoxyliert und/oder polypropoxyliert sind, dass sie ethoxylierte und/oder propopoxylierte W/O-Emulgatoreri darstellen.

Besonders bevorzugt. ist es, wenn der W/O-Emulgator oder die W/O-Emulgatoren so gewählt werden, dass die Reste A und A' werden vorteilhaft gewählt aus der Gruppe der verzweigten und unverzweigten, gesättigten und ungesättigten Alkyl- und Acylreste und Hydroxyacylreste mit 10 - 30 Kohlenstoffatomen sowie ferner aus der Gruppe der über Esterfunktionen miteinander verbundenen Hydroxyacylgruppen, nach dem Schema. 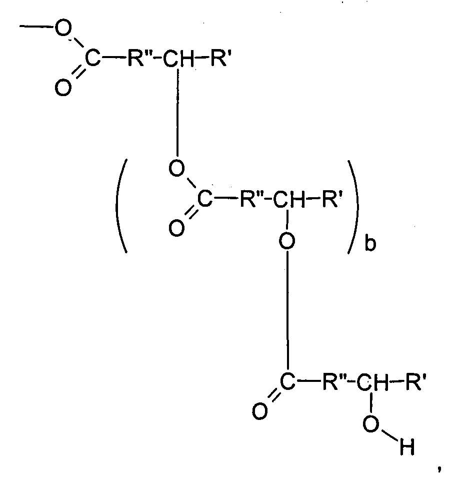 wobei R' gewählt wird aus der Gruppe der verzweigten und unverzweigten Alkylgruppen mit 1 bis 20 Kohlenstoffatomen und R" gewählt wird aus der Gruppe der verzweigten und unverzweigten Alkylengruppen mit 1 bis 20 Kohlenstoffatomen und b Zahlen von 0 bis 200 annehmen kann.

Ganz besonders bevorzugt ist es, wenn der oder die W/O-Emulgatoren gewählt werden aus der Gruppe PEG-30 Dipolyhydroxystearat, Decaglycerylheptaoleat, Polyglyceryl-3-Diisostearat, PEG-8 Distearat, Diglycerin Dipolyhydroxystearat.

Als besonders geeignet hat sich der Einsatz von Emulgatoren im Bereich von 1 bis 8 Gew.% erwiesen. Besonders bevorzugt sind Emulgatorkonzentrationen von 1 bis 3 Gew.-%. Diese Angaben beziehen sich auf das Gesamtgewicht der Emulsion.

Es ist erfindungsgemäß besonders vorteilhaft, wenn das Verhältnis von Hydrokolbiden zu W/O-Emulgatoren 0,4:1 bis 0,8:1 beträgt.

Erfindungsgemäß vorteilhaft ist eine Gesamtpolarität der lipophilen Phase von 10 bis 45 mN/m, wobei besonders vorteilhaft eine Gesamtpolarität der lipophilen Phase von 15 bis 30 mN/m ist.

Des Weiteren können in die erfindungsgemäßen Formulierungen die in der Kosmetik üblichen Hilfs- und Zusatzstoffe eingearbeitet werden, beispielsweise
- Antioxidantien
- Konservierungsmittel
- Parfüm
- Farbstoffe
- Puffersysteme
- Glyceride

Erfindungsgemäß enthalten die Zubernitungen vorteilhaft eines oder mehrere Antioxidantien. Als günstige, aber dennoch fakultativ zu verwendende Antioxidantien können alle für kosmetische und/oder dermatologische Anwendungen geeigneten oder gebräuchlichen Antioxidantien verwendet werden.

Besonders vorteilhaft im Sinne der vorliegenden Erfindung können wasserlösliche Antioxidantien eingesetzt werden, wie beispielsweise Vitamine, z. B. Ascorbinsäure und deren Derivate.

Bevorzugte Antioxidantien sind ferner Vitamin E und dessen Derivate sowie Vitamin A und dessen Derivate.

Die Menge der Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Sofern Vitamin A bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Es ist insbesondere vorteilhaft, wenn die kosmetischen Zubereitungen gemäß der vorliegenden Erfindung kosmetische oder dermatologische Wirkstoffe enthalten, wobei bevorzugte Wirkstoffe Antioxidantien sind, welche die Haut vor oxidativer Beanspruchung schützen können.

Vorteilhafte Konservierungsmillel im Sinne der vorliegenden Erfindung sind beispielsweise Formaldehydabspalter (wie z. B. DMDM Hydantoin, welches beispielsweise unter der Handelsbezeichnung Glydant ™ von der Fa. Lonza erhältlich ist), Iodopropylbutyl-carbamate (z. B. die unter den Handelsbezeichnungen Glycacil-L, Glycacil-S von der Fa. Lonza erhältlichen und/oder Dekaben LMB von Jan Dekker), Parabene (d.h. p-Hydroxybenzoesäurealkylester, wie Methyl-, Ethyl-, Propyl- und/oder Butylparaben), Phenoxyethanol, Ethanol, Benzoesäure und dergleichen mehr. Üblicherweise umfaßt das Konservierungssystem erfindungsgemäß ferner vorteilhaft auch Kopservierungshelfer, wie beispielsweise Octoxyglycerin, Glycine Soja etc.

Als Moisturizer werden Stoffe oder Stoffgemische bezeichnet, welche kosmetischen oder dermatologischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.

Vorteilhafte Moisturizer im Sinne der vorliegenden Erfindung sind beispielsweise Glycerin, Milchsäure und/oder Lactate, insbesondere Natriumlactat, Butylenglykol, Propylenglykol, Biosaccaride Gum-1, Glycine Soja, Ethylhexyloxyglycenn, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z.B. unter der Bezeichnung Fucogel®1000 von der Gesellschaft SOLABIA S.A. erhältlich ist.

Vorteilhaft im Sinne der Erfindung ist ferner die Tränkung von unlöslichen Substraten wie Tüchem, Watte und anderen mit der Ernulsion.

Die erfindungsgemäßen kosmetischen oder dermatologischen Tücher können sowohl aus wasserlöslichen (z. B. wie Toilettenpapier) als auch aus wasserunlöslichen Materialien bestehen. Ferner können die Tücher glatt oder auch oberflächenstrukturiert sein.

Erfindungsgemäß bevorzugt werden "trockene" Tücher ein gesetzt, welche aus Vlies bestehen, insbesondere aus wasserstrahlverfestigten und/oder wasserstrahlgeprägten Vlies.
Derartige erfindungsgemäße Vliese können Makroprägungen jeden gewünschten Musters aufweisen.

Die Dicke des Vlieses beträgt vorzugsweise 0,4 mm bis 2 mm, insbesondere 0,6 mm bis 0,9 mm.

Als Ausgangsmaterialien für den Vliesstoff des Tuches können generell alle organischen und anorganischen Faserstoffe auf natürlicher und synthetischer Basis verwendet werden. Beispielhaft seien Viskose, Baumwolle, Zellulose, Jute, Hanf, Sisal, Seide, Wolle, Polypropylen, Polyester, Polyethylenterephthalat (PET), Aramid, Nylon, Polyvinylderivate, Polyurethane, Polylactid, Polyhydroxyalkanoat,. Celluloseester und/oder Polyethylen sowie auch mineralische Fasern wie Glasfasern oder Kohlenstoffasern angeführt. Die vorliegende Erfindung ist aber nicht auf die genannten Matenalien beschränkt, sondern es können eine Vielzahl weiterer Fasern zur Vliesbildung eingesetzt werden. Es ist insbesondere vorteilhaft im Sinne der vorliegenden Erfindung, wenn die eingesetzten Fasern nicht wasserlöslich sind.

In einer besonders vorteilhaften Ausführungsform des Vlieses bestehen die Fasern aus einer Mischung aus 70 % Viskose und 30 % PET.

Besonders vorteilhaft sind auch Fasern aus hochfesten Polymeren wie Polyamid, Polyester und/oder hochgerecktem Polyethylen.

Darüber hinaus können die Fasern auch eingefärbt sein, um die öptische Attraktivität des. Vlieses betonen und/oder erhöhen zu können. Die Fasern können zusätzlich UV-Stabilsatoren und/oder Konservierungsmittel enthalten.

Es hat sich als vorteilhaft herausgestellt für das Tuch, wenn dieses ein Gewicht von 35 bis 120 g/m², vorzugsweise von 40 bis 60 g/m², hat (gemessen bei 20 °C ± 2 °C und bei einer Feuchtigkeit der Rau mluft von 65 % ± 5 % für 24 Stunden).

Die zur Bildung des Tuches eingesetzten Fasern weisen vorzugsweise eine Wasseraufnahmerate von mehr als 60 mm/[10 min] (gemessen mit dem EDANA Test 10.1-72), insbesondere mehr als 80 mm/[10 min] auf.

Fernen weisen die zur Bildung des Tuches eingesetzten Fasern vorzugsweise ein Wasseraufnahmevermögen von mehr als 5 g/g (gemessen mit dem EDANA Test 10.1-72), insbesondere mehr als 8 g/g auf.

Vorteilhafte Tücher im Sinne der vorliegenden Erfindung haben eine Reißkraft von insbesondere

| | | |
|---|---|---|
| | | [N/50mm] |
| im trockenen Zustand | Maschinenrichtung | >60, vorzugweise >80 |
| | Querrichtung | >20, vorzugsweise >30 |
| im getränkten Zustand | Maschinenrichtung | >4, vorzugsweise >60 |
| | Querrichtung | >10, vorzugsweise >20 |

Die Dehnfähigkeit vorteilhafter Tuches beträgt vorzugsweise

| | | |
|---|---|---|
| | | [N/50mm] |
| im trockenen Zustand | Maschinenrichtung | 15 % bis 100 %, bevorzugt 20 % und 50 % |
| | Querrichtung | 40 % bis 120 %, bevorzugt 50 % und 85 % |
| im getränkten Zustand | Maschinenrichtung | 15 % bis 100 %, bevorzugt 20 % und 40 % |
| | Querrichtung | 40 % bis 120 %, bevorzugt 50 % und 85 % |

Die erfindungsgemäßen kosmetischen und/oder dermatologischen Emulsionen finden Verwendung in Formulierungen zur Gesichtsreinigung, insbesondere zur Entfernung von Make-up, Talkresten und anderen Schmutzpartikeln.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Emulsion bezogen.

### Beispiele

| | **1** | **2** | **3** | **4** | **5** |
|---|---|---|---|---|---|
| PEG-30 Dipolyhydroxystearat | 1,0 | 3,0 | 1,5 | 2,0 | 1,75 |
| Isopropylpalmitat | 3,0 | | | | |
| C12-15 Alkylbenzoat | | 10,0 | | | |
| Octylcocoat | | 10,0 | | 5,0 | |
| Cetiol CC | 4,5 | | | | |
| Cyclomethicon | 5,0 | | | 5,0 | |
| Magnesiumsulfat | | 0,7 | 0,5 | | 0,5 |
| Glycerin | 3,0 | 10,0 | 5,0 | 5,0 | 3,0 |
| Chitosan | 0,25 | | | 1,0 | |
| Biosaccharid Gum | | 1,0 | | | |
| Xanthan Gum | | | 0,5 | | |
| Carbomer | | | | | 0,4 |
| Milchsäure | 0,15 | | | | |
| Phenoxyethanol | 0,4 | 0,3 | | 0,2 | |
| Diazolidinylharnstoff | | | 0,5 | 0,1 | |
| 1,3-Dimethylol-5,5-dimethylhydantoin(DMDM Hydantoin) | 0,1 | | | | 0,5 |
| lodopropynylbutylcarbamat | | | 0,1 | | |
| p-Hydroxybenzoesäurealkylester(Paraben) | 0,4 | 0,1 | | 0,4 | |
| Natriumchlorid | 1,0 | | | 1,0 | |
| Wasser | ad 100 | ad 100 | ad 100 | ad 100 | ad 100 |

## Patentansprüche

1. Kosmetische und/oder dermatologische Wasser-in-Öl-Emulsionen, die auf der Haut aufbrechen, enthaltend
a) eine wässrige Phase mit einem Anteil von mindestens 75 Gewichts-%
b) eine lipophile Phase mit einem Anteil von höchstens 20 Gewichts-% und
c) ein oder mehrere Hydrocolloide aus der Gruppe der Gummen, Polysaccharide, Cellulosederivate, Schichtsilikate, Polyacrylate und/oder anderen Polymeren mit einem Anteil von 0,1 bis 5 Gewichts-%,
d) einen W/O Emulgator oder ein Gemisch aus mehreren W/O Emulgatoren , gewählt aus der Gruppe der grenzflächenaktiven Substanzen der allgemeinen Struktur A-B-A', wobei A und A' gleiche oder verschiedenen hydrophobe organische Reste darstellen und B eine hydrophile Gruppe bedeutet, in einer Konzentration von 1 bis 8 Gewichts-%
jeweils bezogen auf das Gesamtgewicht der Emulsion, mit einem Verhältnis von Hydrocolloiden zu W/O-Emulgatören von 0,25:1 bis 1:1.

2. Kosmetische und/oder dermatologische Wasser-in-ol-Emulsionen nach Anspruch 1, **dadurch gekennzeichnet dass** sie als Hydrokolloid ein Polysaccharid enthalten

3. Kosmetische und/oder dermatologische Wasser-in-Öl-Emulsionen nach einem der Ansprüche 1 oder 2, **dadurch gekennzeichnet dass** die wässrige Phase 80 bis 90 Gewichts-% und die lipophile Phase 10 bis 20 Gewichts-% jeweils bezogen auf das Gesamtgewicht der Emulsion , ausmacht.

4. Kosmetische und/oder dermatologische Wasser-in-Öl-Emulsionen nach einem der Ansprüche 1 bis 3, **dadurch gekennzeichnet dass** die Gesamtpolantät der lipophilen Phase zwischen 10 und 30 mN/m liegt.

5. Kosmetische und/oder dermatologische Wasser-in-Öl-Emulsionen nach einem der Ansprüche 1 bis 4, enthaltend weitere kosmetische und/oder dermatologische Wirk-, Hilfs- und Zusatzstoffe.

6. Tuch oder Watte getränkt mit einer kosmetischen und/oder dermatologischen Wasser-in-ÖI-Emulsion nach einem der Ansprüche 1 bis 5.

7. Verwendung einer kosmetischen und/oder dermatologischen Emulsion nach einem der Ansprüche 1 bis 6 zur Gesichtsreinigung und/oder Make.-Up Entfernung.

8. Verwendung einer kosmetischen und/oder dermatologischen Emulsion nach einem der Ansprüche 1 bis 6 als Hautreinigungsprodukt von hoher Reinigungsleistung, hoher Milde und Hautverträglichkeit, welches ein angenehmes fettfreies Hadtgefühl hinterlässt.
